Europäisches Patentamt

European Patent Office

Office européen des brevets

(19)

(11) EP 0 957 786 B1

(12) **EUROPEAN PATENT SPECIFICATION**

(45) Date of publication and mention
of the grant of the patent:
**26.11.2003 Bulletin 2003/48**

(51) Int Cl.$^7$: **A61B 17/32**, A61B 10/00

(21) Application number: **96931649.6**

(22) Date of filing: **19.09.1996**

(86) International application number:
**PCT/US96/15030**

(87) International publication number:
**WO 97/011646 (03.04.1997 Gazette 1997/15)**

(54) **APPARATUS FOR HARVESTING BONE**

VORRICHTUNG ZUR ENTNAHME VON KNOCHEN

APPAREIL DE PRELEVEMENT D'OS

(84) Designated Contracting States:
**CH DE FR GB IT LI SE**

(30) Priority: **29.09.1995 US 537303**
**25.07.1996 US 686333**

(43) Date of publication of application:
**24.11.1999 Bulletin 1999/47**

(73) Proprietor: **Maxilon Laboratories, Inc.**
**Amherst,NH 03031 (US)**

(72) Inventors:
• **ALTOBELLI, David, E.**
**Hollis, NH 03049 (US)**

• **EBNER, Peter, R.**
**Hollis, NH 03049 (US)**

(74) Representative: **Poulin, Gérard et al**
**BREVALEX**
**3, rue du docteur Lancereaux**
**75008 Paris (FR)**

(56) References cited:
**US-A- 2 876 777**     **US-A- 4 221 222**
**US-A- 5 330 495**

**Description**

The present invention relates to the field of surgery. The invention has particular utility in connection with the removal and collection of bone from the surface of one or more donor sites, and the preparation and placement of the autogenous bone material at a second location in the patient., e.g. for use in grafting bone to osseous deficiencies, such as periodontal and dentoalveolar defects, bone deficiencies around dental implants, and numerous orthopedic applications that require grafting.

Many reconstructive procedures used in medicine and dentistry involve the manipulation and healing of bones. Such procedures may involve changes in the position, orientation, shape and size of skeletal structures. A problem that is commonly encountered during such procedures is a lack of bone graft material. Bone graft material may be used in several applications, such as to fill between sections of bone that have been repositioned, to change surface geometry, or to add bone to an area that is deficient, such as in conjunction with periodontal surgery or dental implants in the patients' jaws.

The need to harvest small bone grafts from intraoral sites has been common in periodontal surgery to restore bone defects around teeth. In the case of dental implant surgery, bone grafts may be needed to augment atrophic alveolar ridges of the maxilla and/or mandible and the sinus floor to increase the dimension of these bone sites to accommodate and totally cover the endosseous portion of implant fixtures. Bone grafts also are used in conjunction with guided tissue regeneration, a technique that uses a membrane to isolate hard tissue from soft tissue sites and potentiate hard tissue healing.

Presently, it is often difficult to harvest adequate amounts of autogenous bone from intraoral sites. Therefore, clinicians often rely on non-autogenous sources of graft material, such as bone from cadaver sources (homologous or allogenic grafts), animal sources (heterogenous or xenogeneic grafts), or synthetic bone substitutes. However, healing of non-autogenous material grafts is not as extensive or predictable as healing of autogeneous bone obtained directly from the patient; plus there is the additional cost of such non-autogenous graft materials which can be significant.

Clinicians use several techniques to remove bone for grafting for intraoral procedures. In one such technique rotary instruments, such as side cutting burrs or trephines, are used to remove a piece or section of cortical bone from a local intraoral site in the maxilla or mandible. The cortical bone is often morsalized into a particulate form, either manually with a rongeur like instrument or in a bone mill. The particulate bone is then combined with blood to form an osseous coagulum, which is then positioned and packed into the osseous defect around the teeth or implant. See Robinson, R.E. "Osseous Coagulum for Bone Induction", J. Periodontology 40:503(1969). Suction devices with filters have been fabricated and manufactured to collect the bone dust from rotary instruments. See Hutchinson, RA "Utilization of an Osseous Coagulum Collection Filter", J. Periodontology 44:668(1973). See also Goldman, et al, "Periodontal Therapy", pp 994-1005, C.V. Mosby Co., (1980); and Haggarty, et al., "Autogeneous Bone Grafts: A Revolution in the Treatment of Vertical Bone Defects", J. Periodontology 42:626(1971). While such techniques are widely used by clinicians, the techniques have limitations, since sites to harvest sections of intraoral bone are limited in number and extent because of limited intraoral access, proximity to tooth roots, nerve structures and sinus cavities, and thin plates of bone.

Other techniques for harvesting bone include using chisels or osteotomes to remove and manually collect shavings from the surface. These instruments must be very sharp and the process is often awkward and time consuming. Other manual instruments such as bone files and rasps also remove bone. However, the efficiency of cutting and the ability to use the removed bone is greatly limited. Another technique is to collect bone dust generated by twist drills or taps used to prepare the sites for implant placement. However, much of the bone material may be lost while the site is being irrigated to cool the cutting instrument. When larger amounts of bone are needed for major reconstructive procedures, other sites such as the hip (anterior or posterior ilium), tibia, ribs, or the calvarium often are used. However, using such other sites necessitates a second surgical site, which may require postoperative hospitalization, and thus is less amenable, e.g. in the case of an out-patient dental procedure.

Various surgical devices have been proposed and/or are in use to harvest bone marrow samples for biopsy or devices such as rongeurs or bone cutters or punches to remove sections or convex edges of bone. Surgical devices also are in use in arthroscopy and endoscopy for cutting or drilling bone or tissue and removing the tissue fragments. Ultrasonic devices to cut bone also are in use; however, such devices require the removal of the irrigant and debris liberated by the apparatus. Each of these methods and/or devices, however, suffers from one or more deficiencies as applied to the collection of bone for grafting.

Yet other patented devices have been proposed; each of these, however, suffers from one or more deficiencies:

U.S. Patent Nos. 5,403,317 and 5,269,785 to Bonutti show a method and apparatus for the percutaneous cutting and removal of tissue fragments from human. The Bonutti device removes the tissue fragments by suction, where it can be collected and then placed elsewhere in the patient from where originally obtained. Bonutti employs a flexible drill, and suction to remove the debris to an externally placed collection reservoir, where it is compressed before being replaced into the patient.

U.S. Patent No. 2,526,662 to Hipps discloses a bone meal extractor apparatus for mechanically removing bone meal from a donor bone site through a small percutaneous site using a drill. The drill shavings, which comprise primarily sub-surface bone, are then evacuated into an open cut that the drill passes through, for collection.

U.S. Patent No. 4,798,213 to Doppelt teaches a device for obtaining a bone biopsy for diagnosis of various bone diseases. The Doppelt device is intended to remove a core of bone using a tubular drill, while maintaining the architecture of the tissue. The sample is obtained from the marrow space and not intended from re-implantation.

U.S. Patent No. 5,133,359 to Kedem shows a hard tissue biopsy instrument in which samples are taken using a rotatably driven hollow needle.

U.S. Patent No. 4,366,822 to Altshuler discloses a method and apparatus for bone marrow cell separation and analysis. The Altshuler apparatus collects bone marrow cells in a filtration chamber on a filter interposed between a needle directed into the bone marrow site and an aspirator or vacuum source, i.e. using negative pressure to withdrawal marrow cells through a needle.

U.S. Patent No. 5,052,411 to Schoolman teaches, a vacuum barrier attachment for shielding the operator of a medical tool from harmful aerosols and blood, etc. created by drilling, sawing types of actions, etc. The Schoolman device requires vacuum and is not intended for harvesting tissue for re-implantation.

U.S. Patent No. 4,722,338 to Wright et al discloses a device instrument for removing bone which uses a shearing action similar to a rongeur to cut bone, with means for collecting fragments of bone as they are removed. The Wright et al device reportedly is used mainly for the removal of projections or edges of bone using a shearing mechanism without the intent of harvesting the bone for grafting.

U.S. Patent No. 4,994,024 to Falk teaches an arthroscopy hook-clippers device that allow the unobstructed removal of tissue or bone with removal of the fragments by suction. The Falk device is intended for arthroscopy applications and with the removal of projections of tissue or bone and not specifically for the harvest of tissue for grafting.

U.S. Patent N° 2,876,777 to Kees et al. discloses a surgical cutting instrument, and more particularly a sub-level cutting tool which is adapted to remove a limited amount of material on each stroke of the tool and to retain the fragments thus removed when the instrument is withdrawn. The instrument is also designed to afford protection against damage to structures surrounding the material which is being removed.

Yet other prior art devices are disclosed in U.S. Patent No. 4,466,429 to Loscher et al and U.S. Patent No. 4,844,064 to Thimsen et al.

The present invention provides an instrument for harvesting bone as defined in claim 1. Other features of the invention are set forth in the dependent claims. It is thus a primary object of the present invention to provide an improved device for removing and harvesting bone or the like, and delivering the bone to a second site, which overcomes the aforesaid and other disadvantages of the prior art. A more specific object of the present invention is to provide an improved device for directly, percutaneously or permucosally removing and collecting bone from one or more donor sites, and for temporarily storing the collected bone and preparing the bone for delivery to a pre-selected recipient site.

The invention is directed to a hand-held surgical instrument for the cutting, removal, and storage of bone surface shavings for use as autogenous bone grafts. The instrument is comprised of a blade mounted in a handle for holding and supporting said blade. The blade has a cutting structure adjacent its distal end in the form of a sharpened loop. The loop's wedge shaped cross-section is defined proximally by a perpendicular curved aperture through the blade, and distally by a ground and honed relief. In the preferred form, the handle cooperates to provide a storage space adjacent the distal end of the blade for receiving harvested bone from the cutting structure. This manual instrument is held at an acute angle to the bone, and with minimal downward pressure, is drawn across the bone surface to cut and collect a thin shaving of bone. The blade is preferably retractable to allow the clinician access to the harvested material. A plunger is incorporated into the handle to serve both as a locking mechanism to secure the blade and as a means to advance and consolidate the bone in the distal aspect of the instrument.

Fig. 1 is a perspective view of an associated instrument embodying the invention.
Fig. 2 shows side (2B), top (2A, 2E, 2F), bottom (2C), and sectional (2D) views of the handle.
Fig. 3. shows side (3B), top (3A), bottom (3C), and sectional (3D) views of the plunger.
Fig. 4 shows top (4A), side (4B), and end (4C) view of the blade.
Fig. 5 shows enlarged top (5A) and sectional (5B) views of the distal (cutting) end of the blade.
Fig. 6. is a diagrammatic illustration of the various angles involved in the cutting operation of the blade.
Fig. 7. illustrates the use of the instrument to collect (7A), mix (7B) and apply (7C) bone shavings.
Figs. 8A-8F show modified versions of handle and bone collection systems.

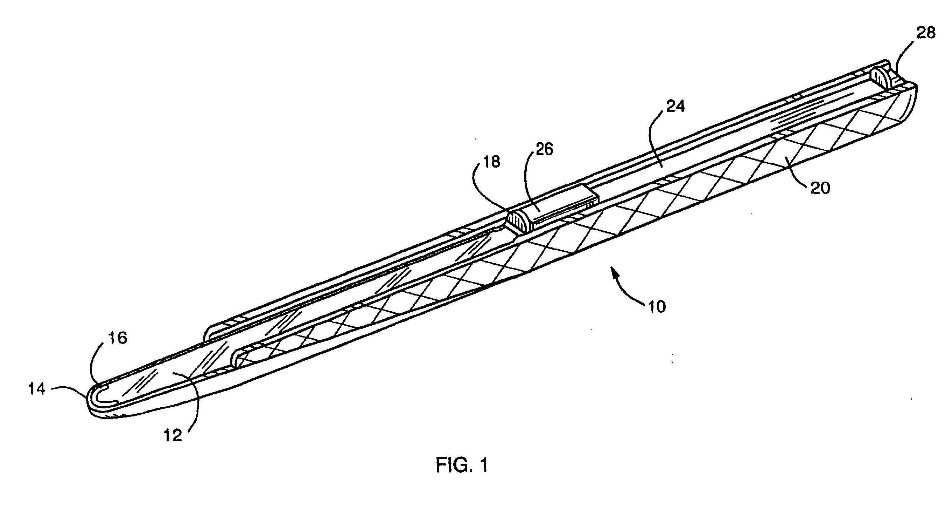

The general arrangement of the elements is shown most clearly in Fig. 1. This shows the assembly comprising the blade 12, the cutting edge 14, and aperture 16, a blade tab 18, the handle 20, a plunger 24, a lock button 26, and a plunger tab 28, all of which are discussed in more detail hereinafter.

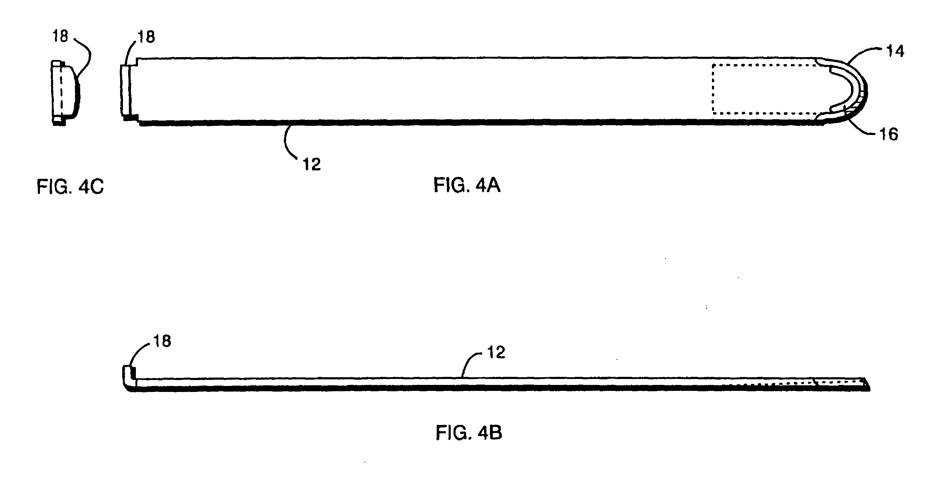

Referring now to Figs. 4, there is shown a construction of a preferred form of the blade of the invention. This cutting structure is comprised of a loop shaped cutting geometry formed on the distal end of the cutting blade 12. The curved structure of the preferred embodiment is a semi-circular cutting edge 14 formed by perforating the distal end of the blade 12 with a semi-circular hole 16. The back surface of the blade, i.e., the surface away from the one adjacent the bone structure, is preferably relieved at 13 between its edges so that the depth of the hole adjacent the cutting edge is equal to or less than the width of the hole 16. This provides easy transfer of the cut bone into the space behind the blade and prevents clogging of the hole during the cutting operation.

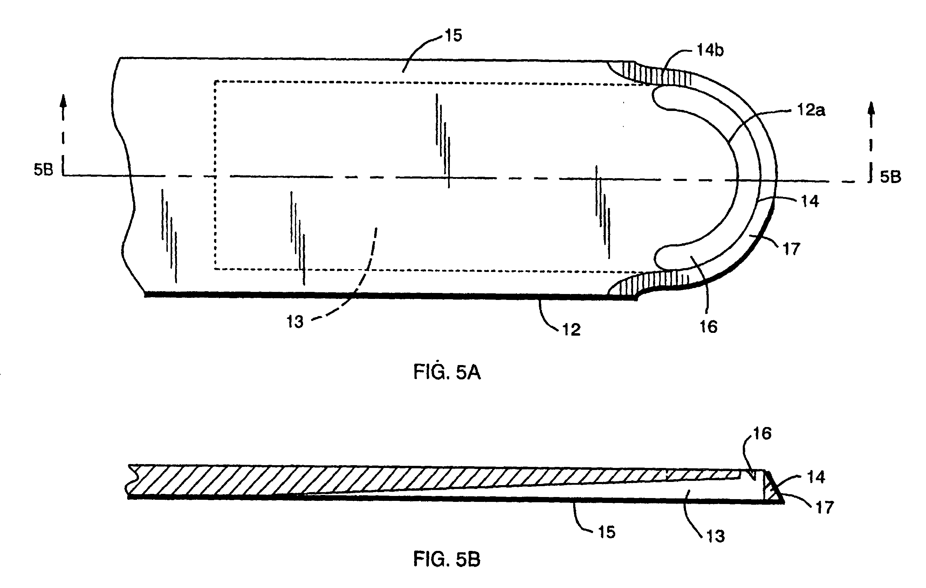

As seen in Fig. 5, the hole 16 in this preferred embodiment is essentially normal to the long dimension of the blade so that the inner side of the cutting edge is essentially normal to the face of the blade which contacts the bone. A slope 17 cooperates with hole 16 to define cutting edge 14. However, in use the blade is held at a slight angle to the bone 100, hence defined the working angle $\alpha_w$. The working angle of the instrument is equivalent to rake angle of the cutting edge with respect to the bone at the tip of the cutting edge 14, with an effective range of positive rake angles from about 5 - 50 degrees when the blade is mounted in the handle.

Novel features of the blade allow manual cutting of the bone, with several advantages over motorized or pneumatic tools. These advantages include decreased costs, decreased set-up time, and decreased heat generation to optimize bone cell survival.

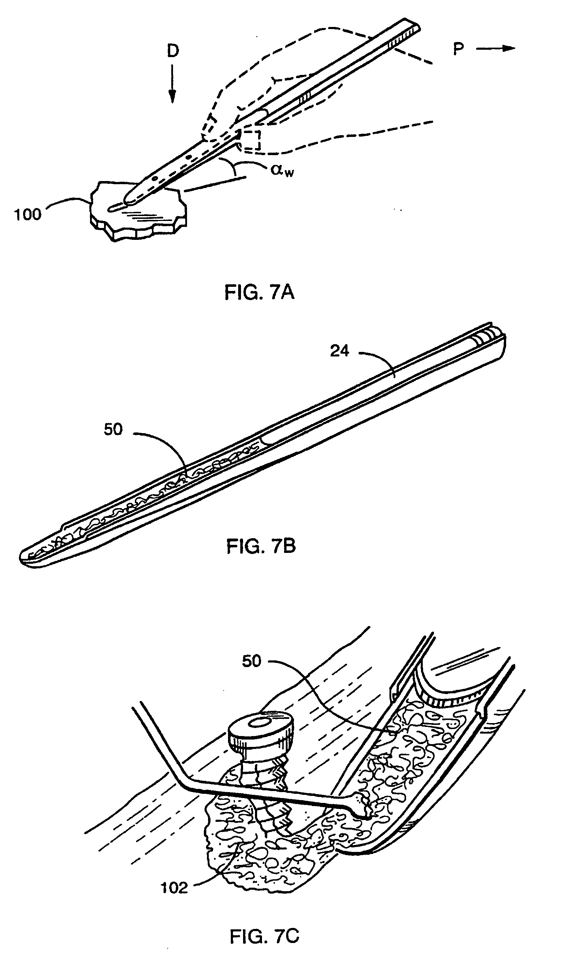

The instrument is easily controlled in comparison to a osteotome or a gouge, where if these instruments disengage from the bone, they lunge into the tissue at the wound borders. With the pulling action of this design as shown as arrow P in Fig. 7A, it is unlikely that the patient would be harmed if the blade inadvertently disengages from the bone. Furthermore, cutting can be carried safely to the boundary of the exposed bone with the blade naturally tracking a straight line without a tendency to veer off.

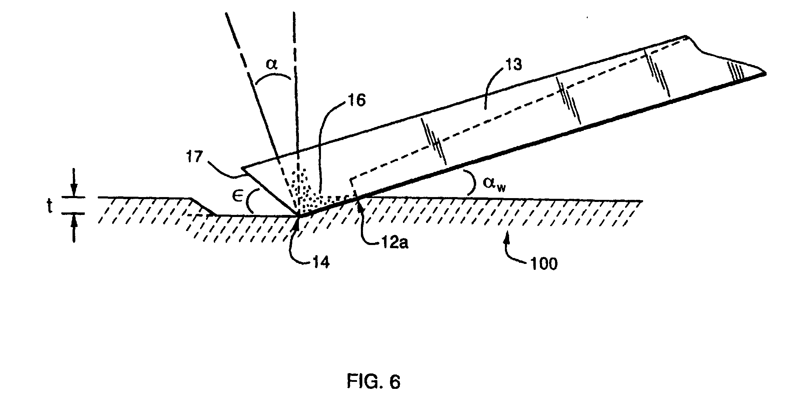

The inner edge of the loop at its distal aspect 14 forms a positive rake angle with respect to the bone surface when the distal end is held in contact with, and at an acute angle to the bone. These various angles are illustrated in Fig. 6. Bone is an anisotropic material with varying requirements for cutting based on orientation. The rake angle ($\alpha$) of the blade edge can be modified by the working angle ($\alpha_w$)of the instrument to the bone surface. This allows adjustment of the cutting parameters of the blade for different bone properties. The Merchant analysis relates the effects of rake angle, depth, and material properties of isotropic materials as a function of a horizontal pulling force (Px) for cutting of a straight blade by:

$$Px = t_0 \ b \ t \cos (\beta - \alpha) \ / \sin \phi \ \cos (\phi + \beta - \alpha)$$

where:

$t_{0 =}$ shear stress at failure on the shear zone (16,260 psi)
$\beta$ = friction angle = arc tan $\mu$ (37°)
$\alpha$ = tool rake angle (5° - 50 °)
$\phi$ = shear plane angle (=34°, 2$\phi$ + $\beta$ + $\alpha$ = 90°)
b = work piece width (estimate 0.020 in)
t = nominal chip thickness (depth of cut, estimate 0.005 in))
$\mu$ = friction coefficient between tool face and chip (0.75)

Substituting values into the equations with a rake angle of 30 degrees :

$$Px = 16,260 \ psi \bullet 0.020in \bullet 0.005in \bullet \cos (37° - 30°) \ / \sin 34° \ \cos$$

$$(34°+37 °-30°)$$

$$= 3.82 \ lbs$$

This relationship is represented graphically for rake angles from 0-55 degrees as is illustrated in graph A below:

## Horizontal Force vs. Rake Angle

GRAPH A

Theoretical results in comparison to experimental results are of limited agreement because of the anisotropic nature of bone. [Jacobs, CH, Pope, MH, Berry, JT, Hoaglund, F. A Study of the Bone Machining Process-Orthogonal Cutting J. Biomechanics, 7:131-136 1974]

As the working angle of the blade's curved loop is increased from zero degrees (full contact of the blade loop with the surface), only the point tangent to the loop's edge 14 remains in contact with the bone surface. Now only slight downward force (shown as arrow D) on the instrument is necessary the cause very high pressures at the interface between the blade and the bone. This allows the blade to penetrate and engage the bone and allows the cut to be initiated. In addition, this point contact allows the blade to engage flat, convex, and most concave bone surfaces. In comparison to a straight or flat blade design where contact surface is not influenced by working angle, contact area is large with higher forces required to penetrate and engage the surface. In addition, cutting of concave surfaces is greatly limited. Approximation of the maximum contact pressure(s) between the blade loop and the bone surface can be estimated from one derivation of the H. Hertz equations for a cylinder on a flat plate of equal modulus :

$$s = 0.591 \ \sqrt{(P_1 E/d_{cyl})}$$

where $P_1 =$ load per inch length, E = modulus of elasticity, $d_{cyl}$ = diameter of cylinder. For a 1 lb. load on the blade edge 0.001 in width and 0.25 in dia, and E for bone $2.61 \times 10^6$ psi :

$$s = 0.591 \ \sqrt{[1000 * 2.61 \times 10^6/0.25]}$$

$$= 60,386 \ psi$$

The modulus of stainless steel is about 10 fold greater than cortical bone, with these contact pressures conservatively less than steel in contact with bone. Cortical bone, with ultimate tensile stress of 20,300 psi, would be indented and engaged by the blade.

Contact stress for a range of blade edge thickness are illustrated below in graph B:

## Edge Width vs Contact Pressure(psi)

**GRAPH B**

The blade allows smooth, uniform cutting of bone with minimal chatter. After engagement of the blade into the bone surface, its positive rake angle further promotes deeper engagement of the blade without increase in normal force. The diving force $F_d$ is a function of the pulling force parallel to the bone surface $F_p$ and the working angle of the instrument $\alpha_w$ :

$$F_d = F_p \sin(\alpha_w)$$

The diving forces increase with the working angle of the instrument. Blade cutting depth reaches equilibrium. This is a function of the laterally decreasing rake angle from the central point of contact, the proximal edge 12A of the blade at the aperture 16, and a wedging effect caused by medial compression of the bone chip as it moves into the circular aperture 16. Geometrically, the aperture width $w_a$ and working angle of the instrument $\alpha_w$ limit the maximum depth of the dive d :

$$d = w_a \sin (\alpha_w)$$

Graphically, with an aperture width of 0.030 in, maximum cutting depth is shown in graph C:

6

**Max. Depth vs Working Angle**

GRAPH C

Anatomic bone surfaces present a terrain of variable contour, with access to these surfaces also limited by adjacent anatomic structures and overlying tissue. The curved loop shaped blade, Fig.5A, provides primary cutting along the longitudinal axis of the instrument. With the cutting edge at the distal end of the blade, this allows access under tissue flaps to the edge of the elevated periosteum. In addition, the blade can be moved laterally at is distal aspect to cut the bone surface in areas of limited access. Cutting now occurs in the more lateral and proximal positions of the loop 19.

The blade edge is hardened to approximately 58 Rockwell C (Brinell hardness~600 kg/mm$^2$) to prolong its cutting life. Cortical bone has harness 80 Rockwell M (Brinell hardness ~30 kg/mm$^2$). Hardness can be further enhanced with titanium nitride coating which also decreases the interface friction between the blade and the bone. The hollow grind of the blade relief at 17 allows the edge profile to be thinner while optimizing the blade stiffness with support above the edge. The edge is both ground and honed in a direction perpendicular to the edge to minimize areas of stress concentration that can occur as the blade thins to its edge.

Bone shavings 50 pass through the narrow aperture 16 and can be collected for eventual grafting purposes. The aperture is analogous to a one way valve, where the shavings easily pass through in their dense form before their shapes and orientations become randomized. This randomized form of the bone is favorable. It prevents the bone shavings from falling back through the aperture 16 and thus not being available for grafting. It allows the shavings to be collected without the use of vacuum which both desiccates and necroses the bone cells, and potentially contaminates the bone with saliva and soft tissue elements.

The bone shavings or chips have favorable properties with respect to their application as autogenous bone grafts. These include an increase in their surface area to volume ratio, an increase in the relative volume of bone, and a porosity that allows incorporation of blood and encourages vascular ingrowth and cell migration into the graft. The exposed collagen promotes coagulation of the blood elements and renders the graft in a favorable "mortar-like" consistency to be packed into the defect sites in the form of an osseous coagulum.

As mentioned previously, the distal end of the upper aspect of the blade incorporates a central ramp or tapered reduced thickness 13, with the proximal end of the ramp decreasing in thickness to where it ends at 12A adjacent the blade aperture 16. This reduced section is shown best in Fig 5B and has the function of providing a very thin blade section 12A immediately adjacent the rear of the hole 16. The blade thickness is maintained laterally with a ridge and sidewalls 15 adjacent to the ramp. This allows the blade loop to have the thickness required for strength, while providing a very short path through the aperture 16. This short passage reduces the chances of clogging.

The ramp 13 serves several additional functions. It provides an initial storage area for the shavings as they are collected. It provides an increase in the cross-sectional area of the reservoir that allows the handles external profile to be reduced in height and thus more accessible to constrained anatomic locations.

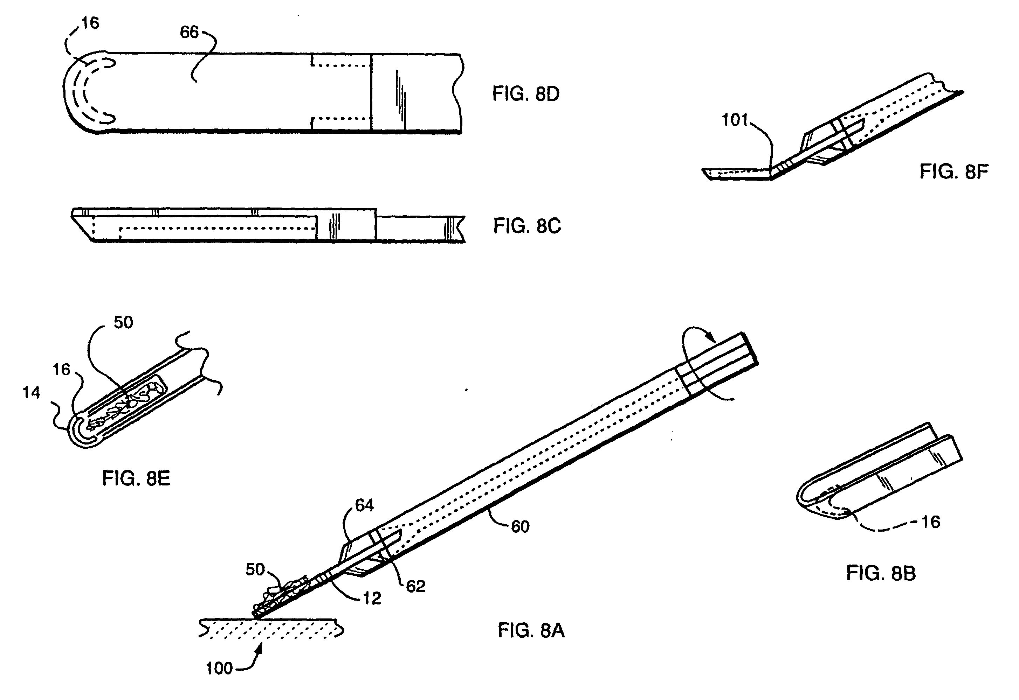

When the handle for the blade does not incorporate the storage area (as shown in Fig. 8a), the ramp 13 provides a seat for the bone as it is collected as shown in figs. 8a-b. A small collection chamber, not integral to the handle, can also be attached to the blade's upper surface as shown in Fig 8c. Finally, in concert with the handle chamber geometry, the chamber is designed with increasing cross-sectional area as one moves proximally, encouraging the chips to move into the handle proximally as the bone is collected.

The blade is preferably bowed longitudinally to create a spring that provides friction between the blade and the grooves 40 of the side wall 30 of the handle 20. This keeps the blade in the desired position until it intentionally needs to be shifted. With the variability in manufacturing, the longitudinal bow geometry allows a relatively large amount of deflection to be used, which makes dimensional variation in production inconsequential. Also, steel , as opposed to the plastic used in the handle, has a predictable modulus of elasticity that will not creep.

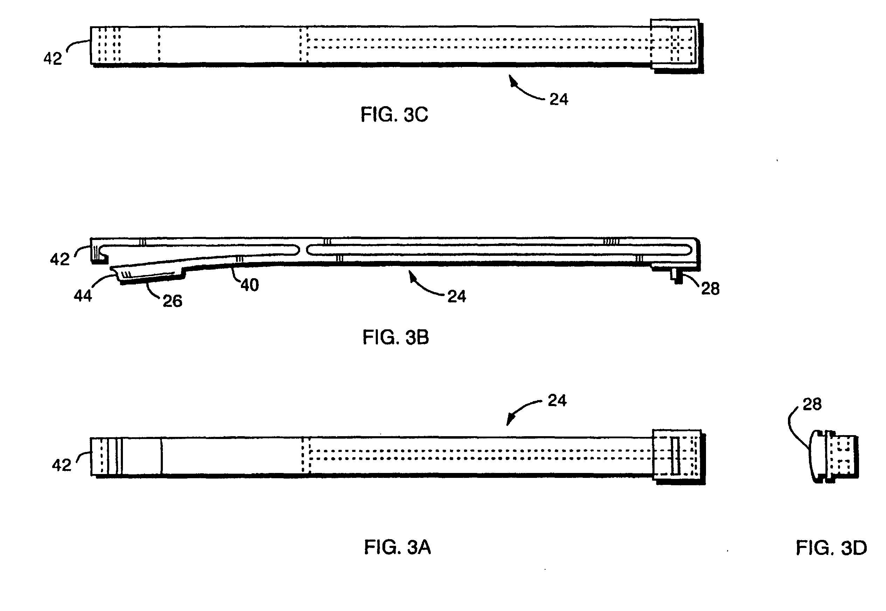

As seen best in Figs. 1 and 4, at the rear of the blade 12 there is provided a blade tab 18 which is adapted to be engaged to a lock button 26 on the end of the plunger 24 (see Figs. 1 and 3) which is mounted in the handle 20 proximal of the blade 12. The extended length of the tab 18 from the cutting edge (as shown in Fig 4A) allows the tab 18 to transmits the raking force from the center of the handle where it has sufficient strength. Furthermore, manipulation of the blade is controlled safely away from the sharp edge. Finally, the extended length blade also serves as a 4th moveable wall of the collection chamber. This allows for a very compact design capable of being used in tight places and allows easy access to the contents of the chamber.

As seen best in Figs. 8A-8E, for constrained anatomic sites, the blade may used with just a gripping handle attached to its proximal end. This leaves the upper surface of the blade exposed on its mid and distal aspects. This allows the blade access to more constrained anatomic locations and also allows the blade to be bent and offset to optimize its access to more specific anatomic locations. The blade can also be increased in thickness to provide more volume in its central ramped hollow to collect the bone shavings.

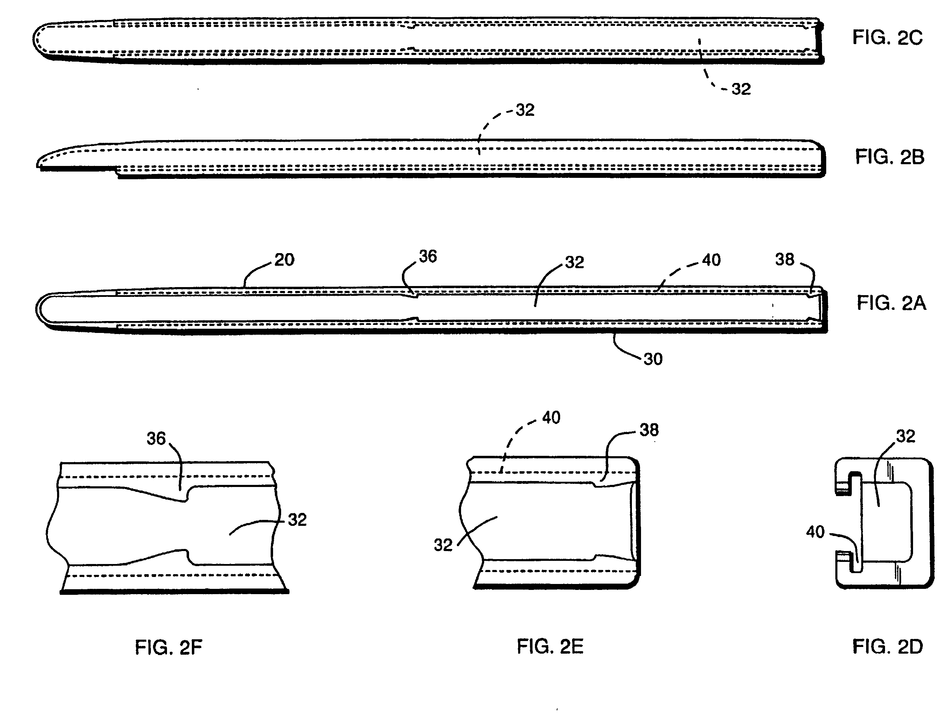

The details of the handle 20 are shown best in Fig. 2 wherein side wall 30 and bottom wall 32 define a u-shaped space 32 as seen best in Fig. 2A. There are two sets of detents, the first set 36 are positioned to engage the tab on the rear end of the blade to prevent its moving beyond the end of the handle 20. The second set 38 are positioned to engage the plunger tab 28 on the plunger 24 and retain the plunger in the position shown Fig. 1 with the lock button 26 in engagement with the blade tab.

The handle of the preferred embodiment serves multiple functions, integrating ergonomic handling and support of the blade, a storage compartment or reservoir of the collected bone, a site for combining additives as needed, and a means of delivering and dispensing the harvested bone at the recipient site. This integrated function also minimizes bone waste and possible contamination by minimizing handling of the bone and the accumulation of the graft material on surfaces such as hoses, filters, containers, etc.

The handle provides safe and clean storage of the harvested bone 50. After passing through the blade aperture 16 the bone enters a closed storage space formed by the handle in conjunction with the blade and the plunger. This space expands in cross-section area as it approaches the proximal aspect of the ramp, encouraging the bone shaves to move into the proximal aspect of the handle. The handle interior provides a trough shaped volume where the contents can be inspected, additives incorporated, and any possible clogging of bone cleared. The plunger 24 can be advanced to consolidate the harvested bone 50 with the blade fully forward. With the blade partially retracted, the plunger advances the graft material to the distal aspect of the handle to provide a streamline trough or channel to deliver bone to the recipient site 102.

The preferred profile for the forward portion of the handle is minimized by transmitting the raking force from the handles center, its thicker and stronger portion. Only a small amount of handle material is required for sufficient strength to carry the normal load to the nose of the handle. This results in a low instrument profile, capable of getting into anatomic spaces of limited dimension. A single pair of grooves 40 guide and retain both the blade and the plunger. This helps to minimize the overall height of the handle.

The handle is preferably fabricated with a clear plastic. This allows the bone shavings to be monitored as they are cut, providing immediate feedback of bone collection. The total volume of bone collected can be monitored with respect to known volume gradations on the handle that inform the surgeon when an adequate volume of bone has been collected.

When the blade is used such that middle and distal portions are exposed, a gripping handle 60 is secured to the proximal end of the blade to facilitate handling of the blade as shown in Figs 8A and 8C-8E. The handle 60 is long and round for secure gripping in the hand and has a slot 62 to accept the blade and a rotatable, tightening mechanism 64 to secure the blade in the handle. Also, as shown in Fig. 8F, the blade may be bent at 101, e.g., to facilitate access to tight spots.

If desired, the blade may be formed into an elongated "cup" shape, i.e. as shown in Fig. 8B, and a clear or transparent cover 66 fitted over the top of the "cup" so as to permit the user to view the progress of bone collection.

The plunger 24 serves two functions; 1) to consolidate and advance the bone into the distal end of the chamber, and 2) it provides a locking mechanism to secure the blade in its forward position.

The plunger head 42 provides the proximal wall of the storage chamber. The plunger is advanced by releasing the locking button 26 which secures both the plunger and the blade in place for cutting and collection. The head of the plunger is held in the track distally by riding under the blade. The proximal end is constrained in the same track 40 that the blade rides in and translates forward to a small stop at the forward end of the handle.

Referring to Fig. 3, the details of the plunger are shown in top view and sectional view wherein the lock button 26 as shown as being mounted on a cantilever arm 46 enabling it to be moved towards the bottom of the plunger. The side edges of the plunger are free of the grooves 40 in the side wall of the handle. The sloping surface 44 on the detent arm 40 engages the proximal end of the blade to press the blade tab 18 against the detents 36, thus locking the blade in correct position.

## Claims

1. An instrument for harvesting bone comprising an elongate body having a proximal end and a distal end, said elongate body serving as a handle (20) for the instrument and securing a blade (12) therein so that the blade can be held at an acute angle with respect to a bone from which bone shavings are to be harvested, said blade (12) having a cutting edge (14), the distal end of the instrument having a curved aperture (16) adjacent the cutting edge of the blade, a tapered curved convex surface forming the distal end of the blade, said blade and handle cooperating to provide a storage space (32) adjacent the distal end of the blade for receiving harvested bone from the cutting structure, said handle (20) being channel-shaped to provide said storage space and said blade serving as a moveable wall of the storage space.

2. The instrument of claim 1, **characterized in that** the handle (20) is of an ergonomic shape which is tapered at least in part to permit access to narrow spaces.

3. The instrument of claim 1, **characterized in that** the moveable wall has a detent (36) for fixing the wall in a closed position.

4. The instrument of claim 1, **characterized in that** the storage space (32) expands in cross-sectional area from the distal to proximal aspect of the handle.

5. The instrument of claim 1, **characterized in that** the handle (20) is formed at least in part of a clear plastic so as to permit visual feedback of progress in collecting bone shavings.

6. The instrument of claim 1, **characterized by** further including a plunger (26) slidably mounted in the handle (20) for consolidating bone shavings in the storage space and for dispensing the consolidated shavings at the distal end of the instrument.

7. The instrument of claim 1, **characterized in that** the blade (12) has a loop shaped cutting structure (14) and the inner edge of the loop at its distal end forms a positive rake angle ($\alpha$) with respect to the bone surface when distal end is held in contact with, and at an angle to, the bone.

## Patentansprüche

1. Instrument zur Knochenentnahme, das umfasst einen länglichen Körper, der ein proximales Ende und ein distales Ende aufweist, wobei der längliche Körper als Haltegriff (20) für das Instrument und zur Fixierung einer Klinge an demselben dient, sodass die Klinge unter einem spitzen Winkel zu einem Knochen gehalten werden kann, aus dem Knochenspäne entnommen werden sollen, wobei die Klinge (12) eine Schneide (14) aufweist, das distale Ende des Instruments eine gekrümmte Apertur (16) aufweist, die der Schneide der Klinge benachbart angeordnet ist und eine sich verjüngende gekrümmte konvexe Oberfläche das distale Ende der Klinge bildet, wobei die Klinge und der Haltegriff zusammenwirken zur Ausbildung eines Aufbewahrungsraums (32) benachbart zu dem distalen Ende der Klinge für die Aufnahme des durch die Schneideeinrichtung entnommenen Knochens, wobei der Haltegriff (20) kanalförmig ausgebildet ist, sodass er einen Aufbewahrungsraum bildet und die Klinge als bewegliche Wand des Aufbewahrungsraumes dient.

2. Instrument nach Anspruch 1, **dadurch gekennzeichnet, dass** der Haltegriff (20) eine ergonomische Gestalt hat, die mindestens zum Teil sich verjüngt, um einen Zugang zu engen Räumen zu ermöglichen.

3. Instrument nach Anspruch 1, **dadurch gekennzeichnet, dass** die bewegliche Wand eine Verriegelung (36) zur Fixierung der Wand in einer geschlossenen Position aufweist.

4. Instrument nach Anspruch 1, **dadurch gekennzeichnet, dass** der Aufbewahrungsraum (32) sich über die Querschnittsfläche des Haltegriffes erstreckt von dem distalen Ende bis zu dem proximalen Ende desselben.

5. Instrument nach Anspruch 1, **dadurch gekennzeichnet, dass** der Haltegriff (20) mindestens zum Teil aus transparentem Kunststoff hergestellt ist, um so eine visuelle Kontrolle des Fortschritts bei der Ansammlung von Knochenspänen zu erlauben.

6. Instrument nach Anspruch 1, **dadurch gekennzeichnet, dass** es außerdem einen Kolben (26) aufweist, der in dem Haltegriff (20) gleitend befestigt ist, für die Konsolidierung der Knochenspäne in dem Aufbewahrungsraum und für die Entnahme der konsolidierten Knochenspäne an dem distalen Ende des Instruments.

7. Instrument nach Anspruch 1, **dadurch gekennzeichnet, dass** die Klinge (12) eine schleifenförmige Schneidestruktur (14) aufweist und der Innenrand der Schleife an seinem distalen Ende einen positiven Spanwinkel (α) gegenüber der Knochenoberfläche bildet, wenn das distale Ende im Kontakt damit und unter einem Winkel gegenüber dem Knochen gehalten wird.

**Revendications**

1. Appareil de prélèvement d'os comprenant un corps allongé ayant une extrémité proximale et une extrémité distale, ledit corps allongé servant de manche (20) à l'appareil et permettant de fixer une lame (12) dans celui-ci de façon à ce que la lame puisse être maintenue en formant un angle aigu par rapport à un os sur lequel des fragments d'os doivent être prélevés, ladite lame (12) ayant un bord tranchant (14), l'extrémité distale de l'appareil ayant une ouverture incurvée (16) adjacente au bord tranchant de la lame, une surface convexe incurvée conique formant l'extrémité distale de la lame, lesdits lame et manche coopérant pour fournir un espace de stockage (32) adjacent à l'extrémité distale de la lame pour recevoir l'os prélevé de la structure de coupe, ledit manche (20) formant un canal pour fournir ledit espace de stockage et ladite lame servant de paroi mobile de l'espace de stockage.

2. Appareil selon la revendication 1, **caractérisé en ce que** le manche (20) a une forme ergonomique qui est conique au moins en partie pour permettre l'accès à des espaces étroits.

3. Appareil selon la revendication 1, **caractérisé en ce que** la paroi mobile possède un cran (36) pour fixer la paroi en position fermée.

4. Appareil selon la revendication 1, **caractérisé en ce que** l'espace de stockage (32) s'étend sur une surface transversale de l'aspect distal à l'aspect proximal du manche.

5. Appareil selon la revendication 1, **caractérisé en ce que** le manche (20) est constitué au moins en partie d'un plastique transparent de façon à permettre un contrôle visuel de la progression du prélèvement de fragments d'os.

6. Appareil selon la revendication 1, **caractérisé en ce qu'**il comprend en outre un piston (26) monté de manière coulissante sur le manche (20) pour pousser les fragments d'os dans l'espace de stockage et pour répandre les fragments poussés jusqu'à l'extrémité distale de l'appareil.

7. Appareil selon la revendication 1, **caractérisé en ce que** la lame (12) a une structure de coupe en forme de boucle (14) et le bord interne de la boucle à son extrémité distale forme un angle de coupe positif (α) par rapport à la surface de l'os lorsque l'extrémité distale est maintenue en contact avec, et en formant un angle par rapport à l'os.

FIG. 1

FIG. 2C

FIG. 2B

FIG. 2A

FIG. 2F

FIG. 2E

FIG. 2D

EP 0 957 786 B1

FIG. 3C

FIG. 3B

FIG. 3A

FIG. 3D

EP 0 957 786 B1

FIG. 4C

FIG. 4A

FIG. 4B

EP 0 957 786 B1

FIG. 5A

FIG. 5B

EP 0 957 786 B1

FIG. 6

FIG. 7A

FIG. 7B

FIG. 7C

FIG. 8F

FIG. 8B

FIG. 8D

FIG. 8C

FIG. 8A

FIG. 8E